Europäisches Patentamt

European Patent Office

Office européen des brevets

(19)

(11) Veröffentlichungsnummer : **0 352 505 B1**

(12) **EUROPÄISCHE PATENTSCHRIFT**

(45) Veröffentlichungstag der Patentschrift :
**09.09.92 Patentblatt 92/37**

(51) Int. Cl.$^5$ : **C07C 37/14, C07C 39/06**

(21) Anmeldenummer : **89112081.8**

(22) Anmeldetag : **01.07.89**

(54) **Verfahren zur Herstellung von Thymol.**

(30) Priorität : **16.07.88 DE 3824284**

(43) Veröffentlichungstag der Anmeldung :
**31.01.90 Patentblatt 90/05**

(45) Bekanntmachung des Hinweises auf die
Patenterteilung :
**09.09.92 Patentblatt 92/37**

(84) Benannte Vertragsstaaten :
**DE FR GB**

(56) Entgegenhaltungen :
**DE-A- 2 242 628**
**DE-A- 2 437 322**
**GB-A- 998 186**
**US-A- 1 886 311**

(56) Entgegenhaltungen :
**SOVIET INVENTIONS ILLUSTRATED, Sektion
Chemie, Woche 8637, Zusammenfassung Nr.
244581, A 60, 26. September 1986, DerwentPublications Ltd., London, GB; & SU-A-12 09678
BULLETIN OF THE CHEMICAL SOCIETY OF
JAPAN, Band 49, Nr. 10, Oktober 1976, Seiten
2669-2674, Tokyo, Japan; T. YAMANA-
KA;"Catalytic Properties of Metal Sulfates
Supported on Gamma-A1203 in the Liquid-
Phase Isopropylation of m-Cresol with Propylene"**

(73) Patentinhaber : **BAYER AG
W-5090 Leverkusen 1 Bayerwerk (DE)**

(72) Erfinder : **Wimmer, Peter, Dr.
Bethelstrasse 10
W-4150 Krefeld (DE)**
Erfinder : **Buysch, Hans-Josef, Dr.
Brandenburger Strasse 28
W-4150 Krefeld (DE)**
Erfinder : **Puppe, Lothar, Dr.
Am Weiher 10A
W-5093 Burscheid (DE)**

EP 0 352 505 B1

## Beschreibung

Die Erfindung betrifft ein Verfahren zur Herstellung von Thymol durch Umsetzung von m-Kresol mit Propen an weit- und mittelporigen Zeolithen.

Thymol besitzt Bedeutung als Duftstoff in der Parfümindustrie, als Antispetikum sowie als Bestandteil pharmazeutischer Produkte. Darüber hinaus ist es bevorzugtes Ausgangsmaterial zur Synthese von Menthol (Ullmanns Enzyklopädie der technischen Chemie, 4. Auflage, Band 20, S. 241 und Band 18, S. 213).

Verfahren zur Alkylierung von m-Kresol mit Propen sind in der Literatur mehrfach beschrieben. Dabei werden neben Thymol im wesentlichen die isomeren Isopropylmethylphenole sowie dialkylierte und polyalkylierte Produkte erhalten (Ullmann, loc. cit., Band 18, S. 204). Nach DE-OS 25 28 303 wird Thymol durch Alkylierung von m-Kresol mit Propen in der Flüssigphase bei 360-365°C und 48-50 bar an aktivierten Aluminiumoxiden herge stellt. Die Selektivität an Thymol im Rohprodukt beträgt 80 %. Reines Thymol wird daraus durch fraktionierte Destillation gewonnen. Als Katalysatoren für die Thymol-Synthese sind auch Calciumoxid (JP 45/15491 (1970)) und Aluminium-m-kresolat (GB 1.227.924) beschrieben. Diese Katalysatoren entfalten jedoch nur bei der Durchführung der Reaktion in Autoklavenreaktoren unter erhöhtem Druck brauchbare Aktivität und Selektivität.

Die DE-AS 18 15 846 beschreibt mit Metallsulfaten imprägnierte Aluminiumoxide als Katalysatoren. Auch hierbei wird in Autoklaven in der flüssigen Phase gearbeitet. Um einen Umsatz von 90-99 % zu erzielen, ist ein dreifacher Überschuß an Propen erforderlich. Die Thymol-Selektivität soll 80-82 % betragen, ist jedoch in dieser DE-AS nicht definiert. In einer vom selben Autor verfaßten Publikation (Bull. Chem. Soc. Japan 49 (1976), 2669) wird ähnliches Datenmaterial vorgelegt. Mit den bereits erwähnten Metallsulfat-Katalysatoren werden gute Selektivitäten nur bei dreifachem Propen-Überschuß und Umsätzen von 5-28 % erzielt. Zur Berechnung der Selektivität werden jedoch nicht sämtliche Nebenprodukte einbezogen. Da bei hohem Propen-Überschuß eine vermehrte Bildung von Nebenprodukten stattfindet, liegen die tatsächlichen Selektivitäten deutlich unter den publizierten Werten.

All diesen bekannten Verfahren ist gemeinsam, daß die Umsetzungen zur Erzielung guter Umsätze und Selektivitäten in der Flüssigphase unter überatmosphärischem Druck durchgeführt werden müssen, da die beschriebenen Katalysatoren unter Normaldruckbedingungen unbefriedigende Wirkungen zeigen. Die damit verbundenen Nachteile ergeben sich aus dem Aufwand, der mit der industriellen Realisierungen der Drucktechnik verbunden ist.

Aus diesem Grund war die Gasphasenpropylierung von m-Kresol unter Normaldruck bereits Gegenstand von Untersuchungen. Mit Metallsulfaten auf Aluminiumoxid- oder Siliziumdioxid-Trägermaterialien werden bei Umsätzen bis zu 42 % maximale Selektivitäten von 70 % erzielt (Bull. Chem. Soc. Japan 47 (1974), 2897). Neben der geringen Ausbeute steht auch die rasche Desaktivierung der beschriebenen Kontakte einer industriellen Anwendung entgegen. In Bull. Chem. Soc. Japan 47 (1974), 2360 ist ein $\gamma$-Al$_2$O$_3$ als Katalysator für die Gasphasenpropylierung von m-Kresol bei Normaldruck beschrieben. Der Umsatz soll 63 % und die Thymol-Selektivität 90 % betragen. Die Selektivitätsberechnung in dieser Publikation bezieht sich nach gegebener Definition nur auf die Thymol-Isomeren. Polyalkylierte Verbindungen, die bis zu 10 % des Reaktionsproduktes ausmachen, gehen nicht in die Kalkulation ein. Dies führt dazu, daß die tatsächliche Selektivität, d.h. der prozentuale Anteil an Thymol, bezogen auf die gesamten Reaktionsprodukte, deutlich geringer ist als die angegebenen Werte. Dies wird durch eigene Vergleichsversuche mit Al$_2$O$_3$-Katalysatoren bestätigt.

Es bestand demnach der Wunsch, ein Verfahren zur Herstellung von Thymol aus m-Kresol und Propen zu entwickeln, das in der Gasphase sowohl bei Normaldruck als auch bei höherem Druck durchgeführt werden kann und das darüber hinaus bei langen Katalysatorstandzeiten gute Umsätze und hohe Selektivitäten ergibt. Aufgrund des Standes der Technik was es überraschend und nicht vorhersehbar, daß die obengenannten Nachteile vermieden werden können, wenn man die Umsetzung an den unten beschriebenen Zeolith-Katalysatoren, die einen Porendurchmesser von mindestens 5 Å besitzen (weit- und mittelporige Zeolithe), durchführt.

Es wurde nun ein Verfahren zur Herstellung von Thymol durch Umsetzung von m-Kresol mit Propen bei erhöhter Temperatur in der Gasphase und normalem bis erhöhtem Druck an heterogenen Katalysatoren gefunden, das dadurch gekennzeichnet ist, daß man als heterogene Katalysatoren weit- und mittelporige Zeolithe, die einen Porendurchmesser von mindestens 5 Å besitzen, einsetzt.

Zeolith-Katalysatoren für das erfindungsgemäße Verfahren fallen unter die Formel

$$M_{m/z}[mMe^1O_2 \cdot nMe^2O_2] \cdot {}_qH_2O \qquad (I),$$

worin
M ein austauschbares Kation bedeutet,
Me$^1$ und Me$^2$ die Elemente des anionischen Gerüstes darstellen,
n/m das Verhältnis der Elemente bedeutet und Werte von 1-3.000, bevorzugt 1-2.000, besonders bevorzugt 1-1.000, annimmt,

z die Wertigkeit des austauschbaren Kations bedeutet und

q die Menge des sorbierten Wassers darstellt.

Zeolithe sind von ihrer Grundstruktur her kristalline Alumosilikate, die aus einem Netzwerk von $SiO_4$- bzw. $AlO_4$-Tetraedern aufgebaut sind. Die einzelnen Tetraeder sind mit Sauerstoffbrücken über die Ecken untereinander verknüpft und bilden ein räumliches Netzwerk, das gleichmäßig von Kanälen und Hohlräumen durchzogen ist. Die einzelnen Zeolith-Strukturen unterscheiden sich durch die Anordnung und Größe der Kanäle und Hohlräume sowie durch ihre Zusammensetzung. Als Ausgleich für die negative Ladung des Gitters sind austauschbare Kationen eingelagert. Die sorbierte Wasserphase $qH_2O$ ist reversibel entfernbar, ohne daß das Gerüst seine Struktur verliert. In (I) ist $Me^1$ im allgemeinen Aluminium, das aber durch andere Elemente ersetzt sein kann, beispielsweise B, Ga, In, Fe, Cr, V, As oder Sb. Weiterhin ist in (I) $Me^2$ hauptsächlich Silizium, das jedoch durch andere vierwertige Elemente ersetzt sein kann, wie beispielsweise durch Ge, Ti, Zr oder Hf. Eine ausführliche Darstellung von Zeolithen ist beispielsweise in der Monographie von D. W. Breck "Zeolite Molecular Sieves, Structure, Chemistry, and Use", J. Wiley & Sons, New York, 1974, gegeben.

Die erfindungsgemäß einsetzbaren Zeolithe sind weit- und mittelporig und haben Porenweiten von mindestens 5 Å, beispielsweise solche im Bereich von 5 bis 9 Å, bevorzugt im Bereich von 5 bis 7 Å.

In bevorzugter Weise kommen Zeolithe der folgenden Strukturtypen für das erfindungsgemäße Verfahren in Frage: Erionit, Faujasit, Zeolith L, Mordenit, Mazzit, Offretit, Zeolith $\Omega$, Gmelinit, Cancrinit, ZSM 12, ZSM 25, Zeolith ß, Ferrierit, ZSM 5, ZSM 11, Heulandit, ZSM 22, ZSM 23, ZSM 48, ZSM 43, ZSM 35, PSH 3, Zeolith $\rho$, ZSM 38, CSZ 1, ZSM 3, ZSM 20 und Chabasit, besonders bevorzugt Mordenit, Zeolith $\Omega$, ZSM 5, ZSM 11, ZSM 23, Zeolith L, Offretit, Erionit und Ferrierit. Ganz besonders bevorzugt sind die Zeolith-Typen Erionit, Mordenit und ZSM 23.

Als austauschbare Kationen können die Zeolithe beispielsweise solche des Li, Na, K, Mg, Cu, Ca, Zn, seltene Erdmetalle, Ti, Zr, Sn, Cr, Fe, Mn, Co, Ni und andere enthalten. Erfindungsgemäß bevorzugt sind solche Zeolithe, bei denen zumindest ein Teil der Metallionen gegen Wasserstoffionen ausgetauscht wurde, bevorzugt 50-100 %, besonders bevorzugt 80-100 %, aller ursprünglich vorhandenen Metallkationen. Die sauren $H^+$-Formen der Zeolithe werden bevorzugt dadurch hergestellt, daß man Metallionen gegen Ammoniumionen austauscht und den aus getauschten Zeolith anschließend kalziniert. Eine weitere Möglichkeit besteht darin, bei Zeolithen, die einen n/m-Wert von 5 oder größer aufweisen, den Protonenaustausch mit Mineralsäuren vorzunehmen. In weiterhin bevorzugter Weise werden daher für das erfindungsgemäße Verfahren die $H^+$-Formen der Zeolithe vom Strukturtyp Mordenit, ZSM 5, ZSM 23, Erionit, Zeolith $\Omega$, Offretit, Zeolith L, ZSM 11 und Ferrierit eingesetzt. Besonders bevorzugt hierunter sind die $H^+$-Formen von Erionit, Mordenit und ZSM 23.

Die Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemäße Verfahren geeignete Form zu bringen. Als Bindermaterialien eignen sich beispielsweise $Al_2O_3$, $SiO_2$ oder Tonmineralien.

m-Kresol und Propen werden in einem Molverhältnis von 0,1-10, bevorzugt 0,2-6, besonders bevorzugt 0,5-2, eingesetzt.

Das erfindungsgemäße Verfahren wird im allgemeinen in der Gasphase bei 1-5, bevorzugt 1-2 bar une einer Temperatur von 150-400°C, bevorzugt 200-300°C, besonders bevorzugt 230-270°C, durchgeführt. Das erfindungsgemäße Verfahren kann aber auch bei erhöhtem Druck, beispielsweise im Bereich etwa von 5-50 durchgeführt werden.

Die Gesamtmenge der Einsatzstoffe wird mit einer Rate von 0,5-10 g/h pro g Katalysator, bevorzugt 3-5 g/h, dem Reaktor zugeführt.

Eine günstige Verfahrensweise zur Durchführung des erfindungsgemäßen Verfahrens besteht darin, m-Kresol aus einer Dosiervorrichtung zunächst in einen Verdampfer zu leiten. Dem entstandenen Dampf wird Propen im gewünschten Molverhältnis zugespeist und die Gasmischung durch ein beheiztes Reaktionsrohr, welches den Katalysator als Festbett enthält, geleitet. Am Reaktorausgang wird das Produktgemisch durch Kühlen auskondensiert. Die Aufarbeitung des Roh-Thymols erfolgt anschließend durch fraktionierte Destillation. Das in der Kopf-Fraktion zurückgewonnene Ausgangsmaterial m-Kresol wird ebenso wie die abgetrennten isomeren Thymole und mehrfach propylierten Nebenprodukte wieder in den Reaktor zurückgeführt. Hierbei werden die isomeren Isopropylmethylphenole zu Thymol isomerisiert sowie mehrfach propylierte Nebenprodukte zu Thymol entalkyliert. Diese Rückführung von unmittelbar nicht gewünschten Reaktionsprodukten stellt eine bevorzugte Variante des erfindungsgemäßen Verfahrens dar.

Die Zeolith-Katalysatoren zeichnen sich durch eine hohe Standzeit aus. Wenn die Aktivität des Katalysators nach längerem Gebrauch langsam abnimmt (beispielsweise durch einsetzende Verkokung), kann er durch Behandlung mit Luft bei einer Temperatur von 250-800°C regeneriert werden; der Katalysator erhält hierbei seine ursprüngliche Aktivität zurück.

Die Zeolith-Katalysatoren zeichnen sich überdies durch eine hohe Selektivität aus. Die bevorzugte Va-

EP 0 352 505 B1

riante der Rückführung der Thymol-Isomeren und mehrfach propylierter Verbindungen führt zu einer weiteren Steigerung der Selektivität an Thymol über das primäre Reaktionsprodukt hinaus.

Beispiele

Beschreibung des Reaktors und der allgemeinen Reaktionsführung

Der Reaktor bestand aus einem Glasrohr von 35 cm Länge und einem Innendurchmesser von 25 mm, das mit einer elektrischen Widerstandsheizung versehen war. 10 g des Katalysatorgranulats, das 30 Gew.-% des Bindermaterials enthielt und eine mittlere Korngröße von 1-2 mm besaß, wurde in die Mitte des Rohres eingebracht. Die Temperatur in der Katalysatorschüttung wurde mit Hilfe eines Thermoelementes gemessen. Die Katalysatorschüttung wurde mit Glaskugeln unter- und überschichtet. Das m-Kresol wurde über eine Dosierpumpe zugeführt und in einer Verdampferzone in die Gasphase übergeführt. Das Propen wurde aus einer Stahlflasche eingeleitet, die zugeführte Gasmenge wurde durch Wägung bestimmt. Die kondensierbaren Reaktionsprodukte wurden in einer Kühlfalle bei 0°C kondensiert. Nach den angegebenen Zeitabschnitten wurden Proben zur gaschromatographischen Analyse entnommen.

Die in den folgenden Beispielen eingesetzten Zeolithe sind durch nachstehende $SiO_2/Al_2O_3$-Verhältnisse charakterisiert:

| Beispiel | Zeolith-Typ | $SiO_2/Al_2O_3$ |
|---|---|---|
| 1a | H-ZSM 5 | 48 |
| 1b | H-ZSM 5 | 326 |
| 2 | H-Offretit | 6,0 |
| 3 | H-Zeolith Ω | 6,4 |
| 4 | H-Erionit | 6,5 |
| 5 | H-Ferrierit | 17,0 |
| 6 | H-ZSM 23 | 85 |
| 7a-f | H-Mordenit | 16 |

Beispiele 1a-7f

Ein gasförmiges Gemisch aus m-Kresol und Propen wurde in die oben beschriebene Apparatur geleitet. Das molare Verhältnis der Reaktanden, die Katalysatorbelastung und die Temperatur wurden, wie in der folgenden Tabelle I erläutert, variiert.

Versuch 7 ist ein Langzeitversuch. Während der Laufzeit wurden das Molverhältnis und der Durchsatz der Reaktanden variiert.

Beispiel 8 (zum Vergleich)

Die Reaktion wurde analog den Beispielen 1a-7f durchgeführt. Das Ergebnis der gaschromatographischen Analyse ist ebenfalls in Tabelle I dargestellt.

4

## Tabelle I

| Nr. | Katalysator | m-Kresol Propen | °C | Durchfluß-rate (g/g/h) | Probenahme nach h | Umsatz (%)[*] | Selektivität (%)[**] |
|-----|-------------|-----------------|-----|------------------------|-------------------|---------------|----------------------|
| 1a | H-ZSM 5 | 1:1 | 250 | 3,5 | 6 | 34 | 75 |
| 1b | H-ZSM 5 | 1:1 | 250 | 3,5 | 3 | 25 | 72 |
| 2 | H-Offretit | 1:1 | 250 | 3,5 | 6 | 30 | 71 |
| 3 | H-Omega | 1:1 | 250 | 3,5 | 2 | 37 | 72 |
| 4 | H-Erionit | 1:1 | 250 | 3,5 | 2 | 36 | 81 |
| 5 | H-Ferrierit | 1:1 | 250 | 3,5 | 5 | 34 | 72 |
| 6 | H-ZSM 23 | 1:1 | 250 | 3,5 | 6 | 45 | 81 |
| 7a | H-Mordenit | 1:1 | 250 | 3,5 | 4 | 44 | 82 |
| 7b | H-Mordenit | 1:1 | 250 | 3,5 | 10 | 44 | 83 |
| 7c | H-Mordenit | 1:1 | 250 | 3,5 | 16 | 44 | 84 |
| 7d | H-Mordenit | 1:2 | 250 | 4,5 | 20 | 53 | 84 |
| 7e | H-Mordenit | 2:1 | 250 | 3,0 | 28 | 30 | 87 |
| 7f | H-Mordenit | 2:1 | 250 | 3,0 | 36 | 30 | 87 |
| 8 | $\gamma$-Al$_2$O$_3$ | 1:1 | 250 | 3,5 | 2 | 18 | 70 |

[*]   bezogen auf m-Kresol

[**]   $\text{Selektivität} = \dfrac{\text{Mol-\% Thymol}}{\text{Mol-\% aller Reaktionsprodukte}} \times 100$

EP 0 352 505 B1

Aus Tabelle I geht die hohe Selektivität der verwendeten Zeolithe, insbesondere des H-Mordenit, des H-Erionit und des H-ZSM 23, hervor. Selbst bei einem ungünstigen Molverhältnis von m-Kresol/Propen = 1:2 tritt keine Verschlechterung der Selektivität gegenüber dem Molverhältnis von 1:1 ein.

Das als Vergleichsversuch herangezogene Beispiel 8 zeigt, daß ein aktiviertes γ-Aluminiumoxid (das als hochaktives Material in vielen technischen Prozessen zum Einsatz kommt) unter den Bedingungen der Erfindung zwar ebenfalls katalytisch wirkt, jedoch mit deutlich geringerem Umsatz bei relativ niedriger Selektivität bezüglich der Bildung von Thymol.

**Patentansprüche**

1. Verfahren zur Herstellung von Thymol durch Umsetzung von m-Kresol mit Propen bei erhöhter Temperatur in der Gasphase und normalem bis erhöhtem Druck an heterogenen Katalysatoren, dadurch gekennzeichnet, daß man als heterogene Katalysatoren weit- und mittelporige Zeolithe, die einen Porendurchmesser von mindestens 5 Å besitzen, einsetzt.

2. Verfahren nach Anspruch 1, dadurch gekennzeichnet, daß die Zeolithe einen Porendurchmesser von 5-9 Å besitzen.

3. Verfahren nach Anspruch 2, dadurch gekennzeichnet, daß die Zeolithe einen Porendurchmesser von 5-7 Å besitzen.

4. Verfahren nach Anspruch 1, dadurch gekennzeichnet, daß als Zeolithe solche des Typs Erionit, Faujasit, Zeolith L, Mordenit, Mazzit, Offretit, Gmelinit, Cancrinit, ZSM 12, ZSM 25, Zeolith ß, Ferrierit, Zeolith Ω, ZSM 5, ZSM 11, Heulandit, ZSM 22, ZSM 23, ZSM 48, ZSM 43, ZSM 35, PSH 3, Zeolith ρ, ZSM 38, CSZ 1, ZSM 3, ZSM 20 und Chabasit eingesetzt werden.

5. Verfahren nach Anspruch 4, dadurch gekennzeichnet, daß als Zeolithe solche des Typs Mordenit, ZSM 5, Zeolith Ω, ZSM 11, ZSM 23, Zeolith L, Offretit, Erionit und Ferrierit eingesetzt werden.

6. Verfahren nach Anspruch 5, dadurch gekennzeichnet, daß als Zeolithe solche des Typs Erionit, Mordenit und ZSM 23 eingesetzt werden.

7. Verfahren nach Anspruch 1, dadurch gekennzeichnet, daß 50-100 % aller austauschbaren Kationen als H$^+$ vorliegen.

8. Verfahren nach Anspruch 7, dadurch gekennzeichnet, daß 80-100 % aller austauschbaren Kationen als H$^+$ vorliegen.

9. Verfahren nach Anspruch 1, dadurch gekennzeichnet, daß die aus dem rohen Reaktionsgemisch isolierten Nebenprodukte in die Reaktion zurückgeführt werden.

10. Verfahren nach Anspruch 1, dadurch gekennzeichnet, daß bei einer Temperatur von 150-400°C, bevorzugt 200-300°C, besonders bevorzugt 230-270°C gearbeitet wird.

**Claims**

1. Process for the preparation of thymol by reaction of m-cresol with propene at elevated temperature in the gas phase and atmospheric to superatmospheric pressure over heterogeneous catalysts, characterized in that the heterogeneous catalysts used are wide- and medium-pored zeolites which have a pore diameter of at least 5 Å.

2. Process according to Claim 1, characterized in that the zeolites have a pore diameter of 5-9 Å.

3. Process according to Claim 2, characterized in that the zeolites have a pore diameter of 5-7 Å.

4. Process according to Claim 1, characterized in that the zeolites used are those of the type erionite, faujasite, zeolite L, mordenite, mazzite, offretite, gmelinite, cancrinite, ZSM 12, ZSM 25, zeolite β, ferrierite, zeolite Ω, ZSM 5, ZSM 11, heulandite, ZSM 22, ZSM 23, ZSM 48, ZSM 43, ZSM 35, PSH 3, zeolite ρ, ZSM 38, CSZ 1, ZSM 3, ZSM 20 and chabasite.

5. Process according to Claim 4, characterized in that the zeolites used are those of the type mordenite, ZSM 5, zeolite Ω, ZSM 11, ZSM 23, zeolite L, offretite, erionite and ferrierite.

6. Process according to Claim 5, characterized in that the zeolites used are those of the type erionite, mordenite and ZSM 23.

7. Process according to Claim 1, characterized in that 50-100 % of all exchangeable cations are present as $H^+$.

8. Process according to Claim 7, characterized in that 80-100 % of all exchangeable cations are present as $H^+$.

9. Process according to Claim 1, characterized in that the by-products isolated from the crude reaction mixture are recycled into the reaction.

10. Process according to Claim 1, characterized in that the reaction is carried out at a temperature of 150-400°C, preferably 200-300°C, particularly preferably 230-270°C.


## Revendications

1. Procédé de préparation du thymol par réaction du m-crésol avec le propène à température élevée en phase gazeuse à pression normale ou sous pression sur des catalyseurs hétérogènes, caractérisé en ce que l'on utilise en tant que catalyseurs hétérogènes des zéolithes à pores larges et moyens, ayant un diamètre de pore d'au moins 5 Å.

2. Procédé selon la revendication 1, caractérisé en ce que les zéolithes ont un diamètre de pore de 5 à 9 Å.

3. Procédé selon la revendication 2, caractérisé en ce que le zéolithes ont un diamètre de pore de 5 à 7 Å.

4. Procédé selon la revendication 1, caractérisé en ce que l'on utilise des zéolithes des types érionite, faujasite, zéolithe L, mordénite, mazzite, offrétite, gmelinite, cancrinite, ZSM 12, ZSM 25, zéolithe β, ferriérite, zéolithe Ω, ZSM 5, ZSM 11, heulandite, ZSM 22, ZSM 23, ZSM 48, ZSM 43, ZSM 35, PSH 3, zéolithe ρ, ZSM 38, CSZ 1, ZSM 3, ZSM 20, et chabasite.

5. Procédé selon la revendication 4, caractérisé en ce que l'on utilise des zéolithes des types mordénite, ZSM 5, zéolithe Ω, ZSM 11, ZSM 23, zéolithe L, offrétite, érionite et ferriérite.

6. Procédé selon la revendication 5, caractérisé en ce que l'on utilise des zéolithes des types érionite, mordénite et ZSM 23.

7. Procédé selon la revendication 1, caractérisé en ce que 50 à 100% de tous les cations échangeables sont à l'état d'ions $H^+$.

8. Procédé selon la revendication 7, caractérisé en ce que 80 à 100% de tous les cations échangeables sont à l'état d'ions $H^+$.

9. Procédé selon la revendication 1, caractérisé en ce que les produits d'accompagnement isolés du mélange de réaction brut sont recyclés à la réaction.

10. Procédé selon la revendication 1, caractérisé en ce que l'on opère à une température de 150 à 400, de préférence de 200 à 300°C et tout spécialement de 230 à 270°C.